# EUROPÄISCHE PATENTANMELDUNG

(11) **EP 3 002 279 A1**
(43) Veröffentlichungstag der Anmeldung: **06.04.2016**
(21) Anmeldenummer: 14187445.3
(22) Anmeldetag: 02.10.2014
(51) Int. Cl.: C07D 249/14

(54) **Verfahren zur Herstellung von 3-(5-Aminotriazolyl)-Sulfid-Derivaten**

(71) Anmelder: Bayer CropScience AG, 40789 Monheim am Rhein (DE)
(72) Erfinder: Riedriech, Matthias, 50735 Köln (DE); Himmler, Thomas, 51519 Odenthal (DE); Ford, Mark James, 61389 Schmitten (DE); Rodefeld, Lars, 51375 Leverkusen (DE)
(74) Vertreter: BIP Patents

(57) **Zusammenfassung**

Die vorliegende Erfindung betrifft ein neues Verfahren zur Herstellung von 3-(5-Aminotriazolyl)-Sulfid-Derivaten der Formel (IV) 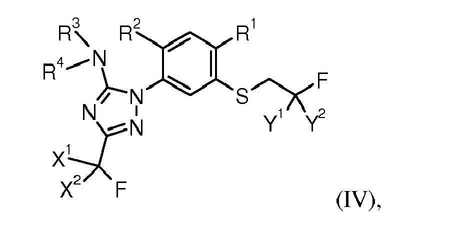 in welcher die Strukturelemente die in der Beschreibung angegebenen Bedeutungen haben.

## Beschreibung

Die vorliegende Erfindung betrifft ein Verfahren zur Herstellung von 3-(5-Aminotriazolyl)-Sulfid-Derivaten der Formel (IV) 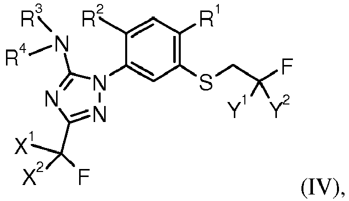 die eine wichtige Vorstufe für 3-(5-Aminotriazolyl)-Sulfid-Derivate der Formel (I) sind.

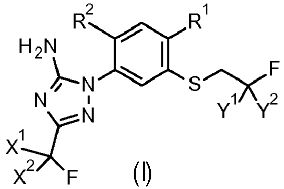

Sulfid-Derivate, insbesondere derartige 3-(5-Aminotriazolyl)-Sulfid-Derivate, haben große technische Bedeutung für die pharmazeutische und agrochemische Industrie und dienen als wichtige und vielfältig einsetzbare Zwischenprodukte für die Herstellung entsprechender Sulfoxid-Derivate, die nachgewiesenermaßen als Pestizide wirken können (WO2009/022548).

Die bisher in der Literatur beschriebenen chemischen Syntheseverfahren von 3-(5-Amino-triazolyl)-Sulfid-Derivaten beinhalten entweder vielstufige lineare Synthesesequenzen und/oder bedienen sich Methoden, die aus industrieller Sicht wirtschaftlich nicht zu realisieren sind. So findet man beispielsweise in WO2006/043635 folgende Syntheseroute zur Herstellung von 3-(5-Aminotriazolyl)-Sulfid-Derivaten.

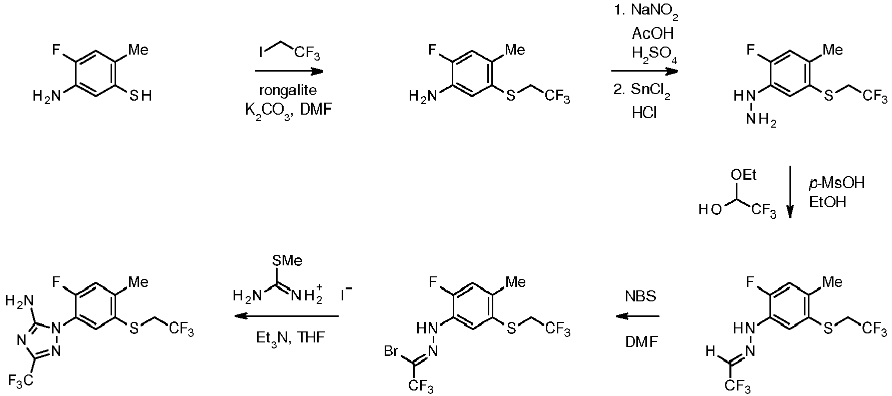

Die Alkylierung des Thiols im ersten Syntheseschritt erfolgt in DMF als Lösungsmittel mit Trifluoriodethan, einem teuren und daher eher zu vermeidenden Reagenz. Die Umwandlung des Anilins in das entsprechende Hydrazin-Derivat im zweiten Syntheseschritt wird unter Zuhilfenahme von SnCl₂ als Reduktionsmittel realisiert. Zinnabfälle müssen wegen ihrer bekannten toxischen Eigenschaften aufwendig entsorgt oder alternativ das entstandene SnCl₄ reduziert und recycelt werden. Für die Bildung des Hydrazons im dritten Syntheseschritt wird das ebenfalls sehr teure 1-Ethoxy-2,2,2-trifluorethanol eingesetzt. Die Bromierung des Hydrazons im vierten Syntheseschritt erfolgt mit ebenfalls teurem N-Brom-Succinimid in DMF als Lösungsmittel. Im fünften und letzten Syntheseschritt dieser Sequenz kommt teures Amino(methyl-sulfanyl)methaniminiumiodid für die Herstellung des Aminotriazols zum Einsatz, wobei iodhaltige Abfälle entstehen. Diese fünf linearen Syntheseschritte und die Verwendung aufwendiger und teurer Reagenzien machen die obige Synthese aus industrieller Sicht unökonomisch.

Eine kürzere, für den industriellen Maßstab aber ebenfalls nicht optimal anwendbare Synthese der 3-(5-Aminotriazolyl)-Sulfid-Derivate wird in WO2009/069312 wie folgt beschrieben.

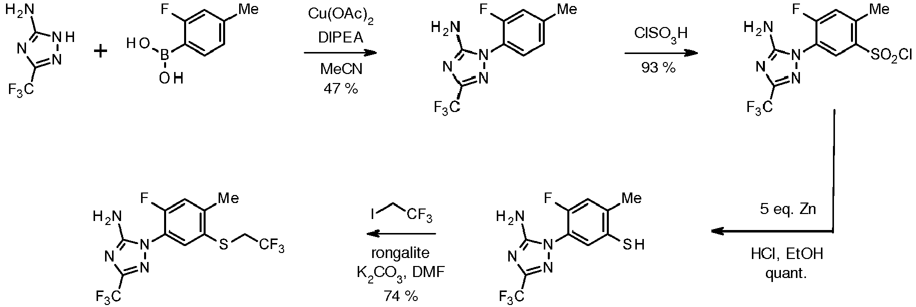

Hier wird das Aryltriazol im ersten Syntheseschritt durch eine kupferkatalysierte Kupplung des 1,2,4-Triazols mit der entsprechenden Boronsäure in einer für industrielle Zwecke zu geringen Ausbeute von 47 % erhalten. Die Herstellung des Sulfonsäurechlorids im zweiten Schritt erfolgt durch die Reaktion des Aromaten mit Chlorsulfonsäure. Das Thiol wird im dritten Syntheseschritt durch Reduktion des Sulfonsäurechlorids mit einem großen Überschuss an Zink erhalten, wobei beträchtliche Mengen zu entsorgender zinkhaltiger Abfälle entstehen. Die Alkylierung des Thiols im vierten und letzten Syntheseschritt erfolgt auch hier mit dem teuren Trifluoriodethan in DMF als Lösungsmittel.

Der Schlüsselschritt der vorliegenden Synthese erfordert u.a. die Bereitstellung der jeweiligen Boronsäurederivate, die sich typischerweise aus den entsprechenden Bromaromaten unter Zuhilfenahme von Butyllithium und Triethylborat in THF bei -70 °C erzeugen lassen (WO99/11620). Bereits bei Temperaturen oberhalb von -20 °C sind insbesondere die (2-Fluorphenyl)boronsäure-Derivate erfahrungsgemäß nur schwer zugänglich. Zudem hängt der Erfolg der oben beschriebenen Kupplung stark von den jeweiligen Reaktionspartnern, dem Heterozyklus und der Boronsäure ab. Eine Übersicht derartiger Transformationen geben beispielsweise J. X. Qiao und P. Y. S. Lam in Synthesis 2011, 6, 829-856 oder K. S. Rao und T-S. Wu in Tetrahedron 2012, 68, 7735-7754. Neben der geringen chemischen Ausbeute im ersten Schritt der Synthesesequenz ist ein weiterer Nachteil der frühe Einsatz des 3-(Trifluormethyl)-1H-1,2,4-triazol-5-amins, das anschließend über drei weitere Syntheseschritte mitgeführt wird. Der Einsatz dieses wertvollen Synthesebausteins zu einem späteren Zeitpunkt der Synthese wäre aus wirtschaftlicher Sicht sinnvoller.

Im Hinblick auf die vorstehend geschilderten Nachteile besteht dringend Bedarf für ein vereinfachtes, technisch und ökonomisch durchführbares Verfahren zur Herstellung von 3-(5-Aminotriazolyl)-Sulfid-Derivaten, insbesondere von substituierten, fluorierten 3-(5-Amino-triazolyl)-Sulfid-Derivaten. Die mit diesem angestrebten Verfahren erhältlichen 3-(5-Amino-triazolyl)-Sulfid-Derivate sollen dabei vorzugsweise mit guter Ausbeute, hoher Reinheit und in ökonomischer Weise erhalten werden.

Prinzipiell lassen sich *N*-Nucleophile mit Halogenaromaten kupferkatalysiert durch Ullmann-Goldberg-Kupplung arylieren (Y. Jiang, D. Ma, G. Evano (Hg.) und N. Blanchard (Hg.) in "Copper-Mediated Cross-Coupling Reactions (1st Edition)" John Wiley & Sons, Inc., Hoboken, New Jersey 2014, 3-40). Während sich für die *N*-Arylierung von Pyrrolen, Pyrazolen und Indazolen, wie von J. C. Antilla et al. in J. Org. Chem. 2004, 69, 5578-5587 beschrieben, sowohl Iod- als auch Bromaromaten eignen, sind die Umsetzungen von Triazolen, insbesondere 1,2,4-Triazolen auf deren Reaktion mit Iodaromaten beschränkt. Die *N*-Arylierung von 5-Amino-Triazolen mit Iodaromaten ist in der Literatur vereinzelt beschrieben (WO2011/049731), für die entsprechende Kupplung mit Iodarylsulfiden liegt bisher keine Präzedenz vor.

Überraschenderweise wurde gefunden, dass in einem Verfahren unter Verwendung eines Kupferkatalysators 3-(5-Aminotriazolyl)-Sulfid-Derivate durch Reaktion eines 5-Amino-Triazol-Derivats mit einem entsprechenden Halogenaromaten hergestellt werden können.

Gegenstand der vorliegenden Erfindung ist demnach ein neues Verfahren zur Herstellung von 3-(5-Aminotriazolyl)-Sulfid-Derivaten der Formel (IV) 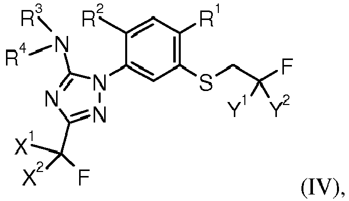 in welcher
X¹, X², Y¹ und Y² unabhängig voneinander für Fluor, Chlor oder Wasserstoff stehen,
R¹ und R² unabhängig voneinander für Wasserstoff, (C₁-C₁₂)Alkyl, (C₁-C₁₂)Halogenalkyl, Cyano, Halogen oder Nitro stehen, und
R³ und R⁴ unabhängig voneinander für Wasserstoff, Trialkylsilyl, Benzyl, *para*-Methoxybenzyl oder gemeinsam für einen Rest #=CHR⁵ stehen, wobei R⁵ für ein ggf. substituiertes Phenyl steht,
dadurch gekennzeichnet, dass ein Halogenaromat der Formel (II) 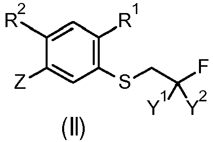
in welcher
Y¹, Y², R¹ und R² die oben genannten Bedeutungen haben und
Z für Chlor, Brom oder Iod steht
mit einem 5-Aminotriazol der Formel (III) 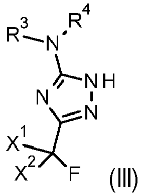
in welcher
X¹,X², R³ und R⁴ die oben genannten Bedeutungen haben,
in Gegenwart eines Kupfer-Katalysators, einer Lewis-Säure und einer Base in einem Lösungsmittel umgesetzt wird.

Bevorzugte, besonders bevorzugte und ganz besonders bevorzugte Bedeutungen der in den vorstehend erwähnten Formeln (IV), (II) und (III) des erfindungsgemäßen Verfahrens aufgeführten Reste X¹, X², Y¹, Y², Z, R¹, R², R³ und R⁴ werden im Folgenden erläutert.

X¹ , X², Y¹ und Y² stehen unabhängig voneinander bevorzugt für Fluor oder Wasserstoff,

Z steht bevorzugt für Brom oder Iod,

R¹ und R² stehen unabhängig voneinander bevorzugt für Fluor, Chlor (C₁-C₃)Alkyl oder Wasserstoff,

R³ und R⁴ stehen unabhängig voneinander bevorzugt für Wasserstoff, Trialkylsilyl, Benzyl oder *para-*Methoxybenzyl.

X¹, X², Y¹ und Y² stehen besonders bevorzugt für Fluor,

Z steht besonders bevorzugt für Brom,

R¹ und R² stehen unabhängig voneinander besonders bevorzugt für Fluor, Wasserstoff oder Methyl,

R³ steht besonders bevorzugt für Wasserstoff,

R⁴ steht besonders bevorzugt für Benzyl oder *para-*Methoxybenzyl.

X¹, X², Y¹ und Y² stehen ganz besonders bevorzugt für Fluor,

Z steht ganz besonders bevorzugt für Brom,

R¹ steht ganz besonders bevorzugt für Fluor,

R² steht ganz besonders bevorzugt für Methyl,

R³ steht ganz besonders bevorzugt für Wasserstoff,

R⁴ steht ganz besonders bevorzugt für *para*-Methoxybenzyl.

Die vorstehend aufgeführten Restedefinitionen bzw. Erläuterungen gelten sowohl für die Endprodukte als auch für die Ausgangsprodukte und Zwischenprodukte entsprechend, insbesondere für die Verbindungen der Formeln (I), (II), (III) und (IV). Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt,.

Erfindungsgemäß ganz besonders bevorzugt werden solche Verbindungen, in welchen eine Kombination der vorstehend als ganz besonders bevorzugt aufgeführten Bedeutungen vorliegt.

Die Verbindungen der Formel (IV) können dann in einem zweiten Schritt in ein 3-(5-Aminotriazolyl)-Sulfid-Derivat der Formel (I) 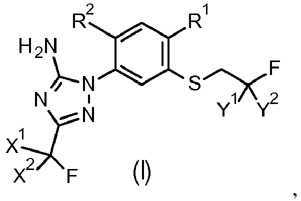 in welcher
X¹, X², Y¹, Y², R¹ und R² die vorstehend genannten Bedeutungen haben, überführt werden.

Dieses ist mittels gängiger Methoden der organischen Chemie möglich (siehe Beispiel 5).

Vorteilhafterweise lassen sich sowohl 3-(5-Aminotriazolyl)-Sulfid-Derivate der Formel (IV) als auch 3-(5-Aminotriazolyl)-Sulfid-Derivate der Formel (I) unter den erfindungsgemäßen Bedingungen mit guten Ausbeuten in hoher Reinheit herstellen.

Das erfindungsgemäße Verfahren kann anhand des folgenden Schemas (I) erläutert werden, welches ferner auch die weitere Überführung von Verbindungen der Formel (IV) in Verbindungen der Formel (I) beinhaltet:

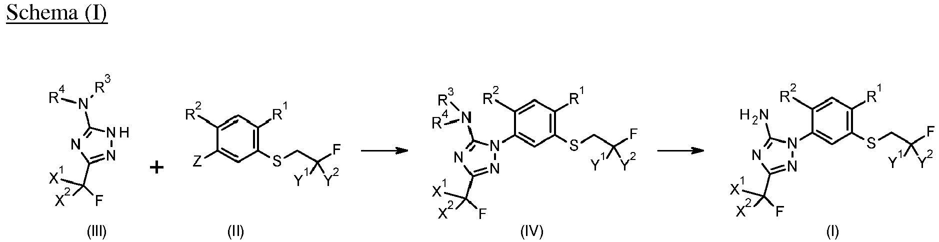

Hierin haben X¹, X², Y¹, Y² , R¹, R², R³ und R⁴ die vorstehend angegebenen Bedeutungen. Das in dem erfindungsgemäßen Verfahren hergestellte 3-(5-Aminotriazolyl)-Sulfid-Derivat der Formel (IV) muss für dessen weitere Überführung in das 3-(5-Aminotriazolyl)-Sulfid-Derivat der Formel (I) nicht notwendigerweise isoliert werden, sondern kann auch *in situ* erzeugt und anschließend direkt zum 3-(5-Aminotriazolyl)-Sulfid-Derivate der Formel (I) umgesetzt werden.

Ein weiterer Gegenstand der vorliegenden Erfindung sind neue Verbindungen der Formel (IIIa) 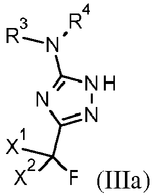 in welcher
X¹ und X² unabhängig voneinander für Fluor, Chlor oder Wasserstoff stehen, und
R³ und R⁴ unabhängig voneinander für Wasserstoff, Trialkylsilyl, Benzyl, *para*-Methoxybenzyl oder gemeinsam für einen Rest #=CHR⁵ stehen, wobei R⁵ für ein ggf. substituiertes Phenyl steht, wobei R³ und R⁴ nicht gleichzeitig Wasserstoff sind.

Bevorzugte, besonders bevorzugte und ganz besonders bevorzugte Bedeutungen der in Formel (IIIa) aufgeführten Reste X¹, X², R³ und R⁴ werden im Folgenden erläutert.

X¹ und X² stehen unabhängig voneinander bevorzugt für Fluor oder Wasserstoff,

R³ und R⁴ stehen unabhängig voneinander bevorzugt für Wasserstoff, Trialkylsilyl, Benzyl oder *para-*Methoxybenzyl, wobei R³ und R⁴ nicht gleichzeitig Wasserstoff sind.

X¹ und X² stehen besonders bevorzugt für Fluor,

R³ steht besonders bevorzugt für Wasserstoff,

R⁴ steht besonders bevorzugt für Benzyl oder *para*-Methoxybenzyl.

X¹ und X² stehen ganz besonders bevorzugt für Fluor,

R³ steht ganz besonders bevorzugt für Wasserstoff,

R⁴ steht ganz besonders bevorzugt für *para*-Methoxybenzyl.

Diese Restedefinitionen können untereinander, also auch zwischen den jeweiligen Vorzugsbereichen, beliebig kombiniert werden.

Erfindungsgemäß bevorzugt werden solche Verbindungen der Formel (IIIa), in welchen eine Kombination der vorstehend als bevorzugt aufgeführten Bedeutungen vorliegt.

Erfindungsgemäß besonders bevorzugt werden solche Verbindungen der Formel (IIIa), in welchen eine Kombination der vorstehend als besonders bevorzugt aufgeführten Bedeutungen vorliegt,.

Erfindungsgemäß ganz besonders bevorzugt werden solche Verbindungen der Formel (IIIa), in welchen eine Kombination der vorstehend als ganz besonders bevorzugt aufgeführten Bedeutungen vorliegt.

Die Verbindungen der Formel (IIIa) können nach bekannten Methoden der organischen Chemie hergestellt und isoliert werden und können im erfindungsgemäßen Verfahren als Ausgangsmaterialien für die Herstellung der 3-(5-Aminotriazolyl)-Sulfid-Derivate der Formel (IV) dienen.

Die Halogenaromaten der Formel (II) können nach bekannten Methoden der organischen Chemie hergestellt und isoliert werden und sind beispielsweise aus WO2007/034755 bekannt.

### Allgemeine Definitionen

Im Zusammenhang mit der vorliegenden Erfindung umfasst der Begriff Halogene (Hal), soweit nicht anders definiert, solche Elemente, die ausgewählt sind aus der Gruppe bestehend aus Fluor, Chlor, Brom und Iod, wobei Fluor, Brom und Iod bevorzugt und Fluor und Brom besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können.

Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Die Kupplung der Halogenaromaten der Formel (II) mit den 5-Aminotriazolen der Formel (III) zu den Verbindungen der Formel (IV) verläuft in Gegenwart eines Kupferkatalysators. Grundsätzlich können alle im Stand der Technik im Zusammenhang mit derartigen Kupplungen beschriebenen Kupferkatalysatoren verwendet werden. Der Kupferkatalysator setzt sich in der Regel aus Kupferpulver (Kupfer(0)) oder einem Kupfer(I)- oder Kupfer(II)-salz und einem Liganden zusammen. Bevorzugte Kupfersalze sind CuI, CuCl, Cu₂O, CuOAc, Cu(OAc)₂ oder CuCl₂, besonders bevorzugte Kupfersalze sind CuI, CuOAc oder Cu(OAc)₂, ganz besonders bevorzugt ist CuOAc oder Cu(OAc)₂.

Der Ligand ist eine Verbindung, die in der Lage ist, mit den Kupfersalzen einen Metall-Ligand-Komplex zu bilden. Solche Liganden werden vorzugsweise ausgewählt aus Verbindungen mit mindestens zwei zur Komplexierung am Metall geeigneten Heteroatomen (beispielsweise O, N, P, S). Bevorzugte Liganden sind solche der Formel (V): 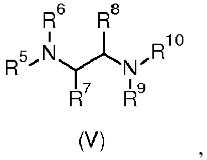 in welcher
R⁵, R⁶, R⁹ und R¹⁰ unabhängig voneinander für Wasserstoff, (C₁-C₆)Alkyl, (C₃-C₆)Cycloalkyl (C₁-C₆)Alkylphenyl oder Phenyl stehen,
R⁷ und R⁸ unabhängig voneinander für Wasserstoff, (C₁-C₆)Alkyl, (C₃-C₆)Cycloalkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
oder gemeinsam für -CH₂-CHR¹¹-CHR¹²-CH₂- stehen, wobei R¹¹ und R¹² unabhängig voneinander für Wasserstoff, (C₁-C₆)Alkyl, (C₃-C₆)Cycloalkyl, (C₁-C₆)Alkylphenyl oder Phenyl stehen.

Eine bevorzugte Verbindung der Formel (V) ist dadurch gekennzeichnet, dass
R⁵, R⁶, R⁹ und R¹⁰ unabhängig voneinander für Wasserstoff oder (C₁-C₆)Alkyl,
R⁷ und R⁸ unabhängig voneinander für Wasserstoff oder (C₁-C₆)Alkyl,
oder gemeinsam für -CH₂-CHR¹¹-CHR¹²-CH₂- stehen, wobei R¹¹ und R¹² unabhängig voneinander für Wasserstoff, (C₁-C₆)Alkyl stehen.

Eine besonders bevorzugte Verbindung der Formel (V) ist dadurch gekennzeichnet, dass
R⁵ und R⁹ für Wasserstoff,
R⁶ und R¹⁰ für (C₁-C₆)Alkyl,
R⁷ und R⁸ für (C₁-C₆)Alkyl,
oder gemeinsam für -CH₂-CHR¹¹-CHR¹²-CH₂- stehen, wobei R¹¹ und R¹² für Wasserstoff stehen.

Eine ganz besonders bevorzugte Verbindung der Formel (V) ist dadurch gekennzeichnet, dass
R⁵ und R⁹ für Wasserstoff,
R⁶ und R¹⁰ für Methyl,
R⁷ und R⁸ gemeinsam für -CH₂-CHR¹¹-CHR¹²-CH₂- stehen, wobei R¹¹ und R¹² für Wasserstoff stehen.

In den Verbindungen der Formel (V) stehen die Reste R⁷ und R⁸ vorzugsweise in trans-Konfiguration zueinander.

Die vorstehend aufgeführten Restedefinitionen bzw. Erläuterungen gelten für die Verbindungen der Formel (V) und können untereinander, also auch zwischen den jeweiligen Vorzugsbereichen, beliebig kombiniert werden.

Erfindungsgemäß bevorzugt werden Verbindungen der Formel (V), in welchen eine Kombination der vorstehend als bevorzugt aufgeführten Bedeutungen vorliegt, vorzugsweise wobei R⁷ und R⁸ in trans-Konfiguration zueinander stehen.

Erfindungsgemäß besonders bevorzugt werden Verbindungen der Formel (V), in welchen eine Kombination der vorstehend als besonders bevorzugt aufgeführten Bedeutungen vorliegt, vorzugsweise wobei R⁷ und R⁸ in trans-Konfiguration zueinander stehen.

Erfindungsgemäß ganz besonders bevorzugt werden Verbindungen der Formel (V), in welchen eine Kombination der vorstehend als ganz besonders bevorzugt aufgeführten Bedeutungen vorliegt, vorzugsweise wobei R⁷ und R⁸ in trans-Konfiguration zueinander stehen.

Bei der Durchführung der Reaktion können die Bestandteile des Katalysatorsystems (Kupfer(0) oder Kupfer(I)- oder Kupfer(II)-salz und Ligand) zusammen oder getrennt zugegeben werden. Die Zugabe erfolgt bei Raumtemperatur oder bei einer Temperatur von 0°C bis 100°C. Bevorzugt erfolgt die Zugabe bei Raumtemperatur. Ebenfalls bevorzugt werden die in der Reaktion verwendeten Kupferkatalysatoren *in situ* aus mindestens einem Kupfersalz und dem entsprechenden Liganden der Formel (V) erzeugt.

Gemäß einer bevorzugten Ausführungsform der vorliegenden Erfindung liegt das molare Verhältnis von Kupfer zu Ligand zwischen 4:1 und 1:50. Gemäß einer besonders bevorzugten Ausführungsform liegt das molare Verhältnis von Kupfer zu Ligand zwischen 1:1 und 1:5. Gemäß einer ganz besonders bevorzugten Ausführungsform liegt das molare Verhältnis von Kupfer zu Ligand zwischen 1:1 und 1:2.

Bezogen auf den Halogenaromaten der Formel (II) beträgt die Einsatzmenge von Kupfer-Ligand-Komplex von 0,001 bis 20 mol%, bevorzugt von 0,1 bis 15 mol% und besonders bevorzugt von 1 bis 10 mol%. Ein höherer Einsatz an Kupfer-Ligand-Komplex ist ebenfalls möglich, aber wirtschaftlich in der Regel nicht sinnvoll.

Gemäß der vorliegenden Erfindung wird zur Kupplung der Halogenaromaten der Formel (II) mit den 5-Aminotriazolen der Formel (III) mindestens eine Lewis-Säure eingesetzt. Erfindungsgemäß können auch Kombinationen von Lewis-Säuren verwendet werden.

Grundsätzlich können alle Lewis-Säuren in dem erfindungsgemäßen Verfahren verwendet werden. In einer bevorzugten Ausführungsform handelt es sich bei der Lewis-Säure um Eisenacetylacetonat (Fe(acac)₃), FeCl₃, FeCl₂, MgCl₂, NiCl₂, ZnCl₂, Zn(OAc)₂, CoCl₂, MnCl₂, VCl₂, AlCl₃, TiCl₄ oder eine Kombination aus einer oder mehreren dieser Säuren. In einer besonders bevorzugten Ausführungsform wird FeCl₃, ZnCl₂, Zn(OAc)₂, MgCl₂ oder AlCl₃ oder eine Kombination aus einer oder mehreren dieser Säuren als Lewis-Säure eingesetzt. Ganz besonders bevorzugt ist ZnCl₂ oder Zn(OAc)₂.

Vorzugsweise wird die Lewis-Säure oder die Kombination von Lewis-Säuren in dem erfindungsgemäßen Verfahren in einer Gesamtmenge von 0,01 bis 1,0 Äquivalenten, vorzugsweise von 0,05 bis 0,5 Äquivalenten und besonders bevorzugt von 0,1 bis 0,3 Äquivalenten, bezogen auf den Halogenaromaten der Formel (II), eingesetzt.

Gemäß der vorliegenden Erfindung wird zur Kupplung der Halogenaromaten der Formel (II) mit den 5-Aminotriazolen der Formel (III) mindestens eine Base eingesetzt. Erfindungsgemäß können auch Kombinationen von Basen verwendet werden.

Grundsätzlich können alle anorganischen oder organischen Basen verwendet werden. In einer bevorzugten Ausführungsform handelt es sich bei der Base um NaHCO₃, KHCO₃, Na₂CO₃, K₂CO₃, Cs₂CO₃, K₃PO₄, NaOAc, KOAc, NaOH, KOH, 1,8-Diazabicyclo[5.4.0]undec-7-en (DBU) oder eine Kombination aus einer oder mehreren dieser Basen. In einer besonders bevorzugten Ausführungsform wird K₂CO₃ oder K₃PO₄ als Base eingesetzt.

Vorzugsweise wird die Base oder die Kombination von Basen in dem erfindungsgemäßen Verfahren in einer Gesamtmenge von 0,1 bis 10,0 Äquivalenten, vorzugsweise von 0,5 bis 5 Äquivalenten und besonders bevorzugt von 1,0 bis 2,5 Äquivalenten, bezogen auf den Halogenaromaten der Formel (II), eingesetzt.

Die Kupplung der Halogenaromaten der Formel (II) mit den 5-Aminotriazolen der Formel (III) zu den Verbindungen der Formel (IV) erfolgt in Gegenwart eines Lösungsmittels. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Acetonitril, Butyronitril, Toluol, Xylole, Mesitylen, Ethylacetat, Isopropylacetat, Butylacetat, Pentylacetat, Methylisobutylketon; Alkohole wie Methanol, Ethanol, Propanol, Butanol, Ethylenglycol; Ethylencarbonat, Propylencarbonat, N,N-Dimethylacetamid, N,N-Dimethylformamid, N-Methylpyrrolidon; Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid, 4-Chlorbenzotrifluorid, und Wasser. Es können auch Lösungsmittelgemische, vorzugsweise Gemische aus den vorstehend genannten Lösungsmitteln, eingesetzt werden.

Bevorzugte Lösungsmittel sind THF, 1,4-Dioxan, Diglyme, Methyltertbutylether (MTBE), tert-Amyl-methylether (TAME), 2-Methyl-THF, Acetonitril, Butyronitril, Toluol, 4-Methoxybenzol und Isopropylacetat.

Besonders bevorzugte Lösungsmittel sind 1,4-Dioxan, 2-Methyl-THF, und 4-Methoxybenzol.

Das Lösungsmittel kann ferner entgast (sauerstoff-frei) sein.

Die Umsetzung wird im allgemeinen bei einer Temperatur zwischen - 80°C und 200°C, vorzugs-weise zwischen 0°C und 150°C, ganz besonders bevorzugt zwischen 20°C und 120°C durchgeführt.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (IV) kann beispielsweise durch anschließende Extraktion und Kristallisation erfolgen. Solche Verfahren sind dem Fachmann bekannt und schließen insbesondere die bevorzugte Kristallisation aus einem organischen Lösungsmittel oder einem Gemisch aus organischem Lösungsmittel mit Wasser ein.

Die vorliegende Erfindung wird anhand der nachfolgenden Beispiele näher erläutert, wobei die Beispiele nicht in einer die Erfindung einschränkenden Weise zu interpretieren sind.

### Herstellungbeispiele:

### Beispiel 1: Synthese von N-Benzyl-3-(trifluormethyl)-1H-1,2,4-triazol-5-amin

In einem 1000 mL Reaktionsgefäß wurden 63,3 g 3-(Trifluormethyl)-1H-1,2,4-triazol-5-amin in 600 mL Toluol gelöst und bei Raumtemperatur mit 41,9 g Benzaldehyd versetzt. Die Reaktionsmischung wurde am Wasserabscheider unter Rückfluss für 14 h gerührt und anschließend auf Raumtemperatur gekühlt. Der gebildete farblose Feststoff (Imin) wurde abfiltriert, ein mal mit 100 mL Toluol gewaschen und im obigen 1000 mL Reaktionsgefäß in 600 mL Ethanol gelöst. Dieser Lösung wurden 22,4 g Natriumborhydrid portionsweise innerhalb von 1 h bei Raumtemperatur zugegeben. Die Reaktionslösung wurde 14 h bei Raumtemperatur gerührt und anschließend mit eiskaltem Wasser versetzt. Der gebildete Feststoff wurde mittels Filtration von der Produktlösung abgetrennt, das Filtrat wurde mit gesättigter NaCl-Lösung und Ethylacetat versetzt. Nach Phasentrennung wurde die wässrige Phase ein mal mit Ethylacetat extrahiert. Die vereinigten organischen Phasen wurden ein mal mit gesättigter NaCl-Lösung gegengewaschen, mit Natriumsulfat getrocknet, filtriert und unter vermindertem Druck am Rotationsverdampfer bis zur Trockne eingeengt. Das Produkt wurde anschließend an der Luft auf einer Tonplatte vollständig getrocknet. Auf diese Weise konnten 35,9 g an *N*-Benzyl-3-(trifluormethyl)-1H-1,2,4-triazol-5-amin in einer Ausbeute von 38,1 % (über zwei Stufen) und einer Reinheit von 99,7 % als farbloser Feststoff isoliert werden.

¹*H*-NMR (600 MHz, DMSO-*d*₆): δ = 12,95 (bs, 1H); 7,59 (t, 1H); 7,35-7,32 (m, 4H); 7,27-7,24 (m, 1H); 4,38 (d, 2H). *Tₘ* = 171 °C.

### Beispiel 2: Synthese von N-(4-Methoxybenzyl)-3-(trifluormethyl)-1H-1,2,4-triazol-5-amin

In einem 1000 mL Reaktionsgefäß wurden 80,0 g 1-(4-Methoxyphenyl)-*N*-[3-(trifluormethyl)-1H-1,2,4-triazol-5-yl]-methanimin in 800 mL Ethanol gelöst und bei Raumtemperatur portionsweise innerhalb von 1 h mit 17,1 g Natriumborhydrid versetzt. Die Reaktionslösung wurde 14 h bei Raumtemperatur gerührt und anschließend mit eiskaltem Wasser versetzt. Das trübe Reaktionsgemisch wurde unter vermindertem Druck am Rotationsverdampfer eingeengt und mit weiterem Wasser versetzt. Der gebildete Feststoff wurde abfiltriert, mit wenig Wasser gewaschen und an der Luft getrocknet (Produktcharge 1: 24,4 g). Die Mutterlauge wurde drei mal mit Ethylacetat extrahiert. Die vereinigten organischen Phasen wurden ein mal mit gesättigter NaCl-Lösung gegengewaschen, mit Natriumsulfat getrocknet, filtriert und unter vermindertem Druck am Rotationsverdampfer bis zur Trockne eingeengt (Produktcharge 2: 49,0 g). Auf diese Weise konnten 73,4 g an N-(4-Methoxybenzyl)-3-(trifluor-methyl)-1H-1,2,4-triazol-5-amin in einer Ausbeute von 89,0 % und einer Reinheit von 97,8 % als farbloser Feststoff isoliert werden.

¹*H*-NMR (600 MHz, DMSO-*d*₆): δ = 13,00 (bs, 1H); 7,49 (t, 1H); 7,26 (d, 2H); 6,90-6,88 (m, 2H); 4,29 (d, 2H); 3,72 (s, 3H). *Tₘ* = 182 °C.

### Beispiel 3: Synthese von N-Benzyl-1-{2-fluor-4-methyl-5-[(2,2,2-trifluorethyl)sulfanyl]-phenyl}-3-(trifluormethyl)-1H-1,2,4-triazol-5-amin

In einem 250 mL Reaktionsgefäß wurden 10,0 g 5-Brom-4-fluor-2-methylphenyl-2,2,2-trifluor-ethylsulfid zusammen mit 12,4 g *N*-Benzyl-3-(trifluormethyl)-1H-1,2,4-triazol-5-amin, 0,59 g Cu(OAc)₂, 1,33 g ZnCl₂, 13,5 g K₂CO₃ und 0,96 g *trans*-*N*,*N'*-Dimethylcyclohexan-1,2-diamin in 100 mL 1,4-Dioxan (entgast) 24 h unter Rückfluss gerührt. Anschließend wurde die Reaktionsmischung bei Raumtemperatur mit je 50 mL Wasser und Toluol versetzt und die Phasen getrennt. Die wässrige Phase wurde zwei mal mit je 30 mL Toluol extrahiert und die vereinigten organischen Phasen mit je 20 mL 17 %-iger Ethylendiaminlösung, Wasser und gesättigter NaCl-Lösung gegengewaschen. Die organische Phase wurde anschließend mit Na₂SO₄ getrocknet und unter vermindertem Druck am Rotationsverdampfer auf ca. 20 mL eingeengt. Durch Zugabe von 100 mL Methylcyclohexan wurde das Produkt zur Kristallisation gebracht und abfiltriert. Auf diese Weise konnten 8,0 g an *N*-Benzyl-1-{2-fluor-4-methyl-5-[(2,2,2-trifluorethyl)sulfanyl]-phenyl}-3-(trifluormethyl)-1H-1,2,4-triazol-5-amin in einer Ausbeute von 50,2 % und einer Reinheit von 95,5 % als schwach brauner Feststoff isoliert werden.

¹*H*-NMR (600 MHz, DMSO-*d*₆): δ = 7,86 (d, 1H); 7,74 (t, 1H); 7,51 (d, 1H); 7,33-7,30 (m, 4H); 7,27-7,23 (m, 1H); 4,46 (d, 2H); 4,07 (q, 2H); 2,45 (s, 3H). *Tₘ* = 81 °C.

### Beispiel 4: Synthese von 1-{2-Fluor-4-methyl-5-[(2,2,2-trifluorethyl)sulfanyl]phenyl}-N-(4-methoxybenzyl)-3-(trifluormethyl)-1H-1,2,4-triazol-5-amin

In einem 250 mL Reaktionsgefäß wurden 10,0 g 5-Brom-4-fluor-2-methylphenyl-2,2,2-trifluor-ethylsulfid zusammen mit 9,5 g *N*-(4-Methoxybenzyl)-3-(trifluormethyl)-1H-1,2,4-triazol-5-amin, 0,58 g Cu(OAc)₂, 1,31 g ZnCl₂, 8,8 g K₂CO₃ und 0,94 g *trans*-*N,N'*-Dimethylcyclohexan-1,2-diamin in 100 mL 1,4-Dioxan (entgast) 24 h unter Rückfluss gerührt. Anschließend wurde die Reaktionsmischung bei Raumtemperatur mit je 50 mL Wasser und Toluol versetzt und die Phasen getrennt. Die wässrige Phase wurde zwei mal mit je 30 mL Toluol extrahiert und die vereinigten organischen Phasen mit je 20 mL 17 %-iger Ethylendiaminlösung, Wasser und gesättigter NaCl-Lösung gegengewaschen. Die organische Phase wurde anschließend mit Na₂SO₄ getrocknet und unter vermindertem Druck am Rotationsverdampfer auf ca. 20 mL eingeengt. Durch Zugabe von 100 mL Methylcyclohexan wurde das Produkt zur Kristallisation gebracht und abfiltriert. Auf diese Weise konnten 11,9 g an 1-{2-Fluor-4-methyl-5-[(2,2,2-trifluorethyl)-sulfanyl]phenyl}-*N*-(4-methoxybenzyl)-3-(trifluormethyl)-1H-1,2,4-triazol-5-amin in einer Ausbeute von 71,5 % und einer Reinheit von 95,4 % als farbloser Feststoff isoliert werden.

¹*H*-NMR (600 MHz, DMSO-*d*₆): δ = 7,83 (d, 1H); 7,66 (t, 1H); 7,50 (d, 1H); 7,23 (d, 2H); 6,89-6,86 (m, 2H); 4,38 (d, 2H); 4,05 (q, 2H); 3,72 (s, 3H); 2,44 (s, 3H). *Tₘ* = 73 °C.

### Beispiel 5: Synthese von 1-{2-Fluor-4-methyl-5-[(2,2,2-trifluorethyl)sulfanyl]phenyl}-3-(trifluormethyl)-1H-1,2,4-triazol-5-amin

In einem 50 mL Einhalskolben wurden 3,00 g 1-{2-Fluor-4-methyl-5-[(2,2,2-trifluorethyl)-sulfanyl]phenyl}-N-(4-methoxybenzyl)-3-(trifluormethyl)-1H-1,2,4-triazol-5-amin vorgelegt und bei Raumtemperatur mit 6,7 mL Trifluoressigsäure versetzt. Die Reaktionsmischung wurde für 5 h bei Raumtemperatur gerührt und anschließend mit 15 mL Toluol versetzt. Das Lösungsmittel und die überschüssige Trifluoressigsäure wurden unter vermindertem Druck am Rotationsverdampfer bis zur Trockne eingeengt. Das Rohprodukt wurde mit Toluol umkristallisiert. Auf diese Weise konnten 1,96 g an 1-{2-Fluor-4-methyl-5-[(2,2,2-trifluorethyl)sulfanyl]phenyl}-3-(trifluormethyl)-1H-1,2,4-triazol-5-amin in einer Ausbeute von 89,6 % und einer Reinheit von 99,1 % als farbloser Feststoff isoliert werden.

¹*H*-NMR (600 MHz, DMSO-*d*₆): δ = 7,80 (d, 1H); 7,48 (d, 1H); 6,99 (s, 2H); 4,05 (q, 2H); 2,45 (s, 3H). *Tₘ* = 180 °C.

## Patentansprüche

1. Verfahren zur Herstellung von 3-(5-Aminotriazolyl)-Sulfid-Derivaten der Formel (IV) 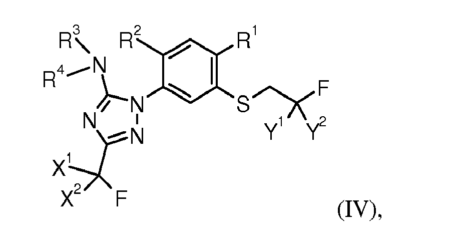 in welcher
X¹, X², Y¹ und Y² unabhängig voneinander für Fluor, Chlor oder Wasserstoff stehen,
R¹ und R² unabhängig voneinander für Wasserstoff, (C₁-C₁₂)Alkyl, (C₁-C₁₂)Halogenalkyl, Cyano, Halogen oder Nitro stehen, und
R³ und R⁴ unabhängig voneinander für Wasserstoff, Trialkylsilyl, Benzyl, *para*-Methoxybenzyl oder gemeinsam für einen Rest #=CHR⁵ stehen, wobei R⁵ für ein ggf. substituiertes Phenyl steht,
**dadurch gekennzeichnet, dass** ein Halogenaromat der Formel (II) 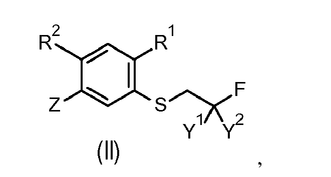 in welcher
Y¹, Y², R¹ und R² die oben genannten Bedeutungen haben und
Z für Chlor, Brom oder Iod steht,
mit einem 5-Aminotriazol der Formel (III) 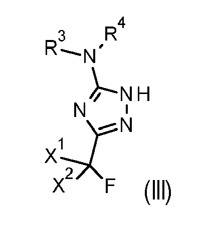 in welcher
X¹,X² , R³ und R⁴ die oben genannten Bedeutungen haben,
in Gegenwart eines Kupferkatalysators, einer Lewis-Säure und einer Base in einem Lösungsmittel umgesetzt wird.

2. Verfahren nach Anspruch 1, **dadurch gekennzeichnet, dass**
X¹, X², Y¹ und Y² unabhängig voneinander für Fluor oder Wasserstoff stehen,
Z für Brom oder Iod steht,
R¹ und R² unabhängig voneinander für Fluor, Chlor (C₁-C₃)Alkyl oder Wasserstoff stehen, und
R³ und R⁴ unabhängig voneinander für Wasserstoff, Trialkylsilyl, Benzyl oder *para-*Methoxybenzyl stehen.

3. Verfahren nach Anspruch 1 oder 2, **dadurch gekennzeichnet, dass**
X¹, X², Y¹ und Y² für Fluor stehen,
Z für Brom steht,
R¹ und R² unabhängig voneinander für Fluor, Wasserstoff oder Methyl stehen,
R³ für Wasserstoff steht, und
R⁴ für Benzyl oder *para*-Methoxybenzyl steht

4. Verfahren nach Anspruch 1 bis 3, **dadurch gekennzeichnet, dass**
X¹, X², Y¹ und Y² für Fluor stehen,
Z für Brom steht,
R¹ für Fluor steht,
R² für Methyl steht,
R³ für Wasserstoff steht, und
R⁴ für *para*-Methoxybenzyl steht.

5. Verfahren nach Anspruch 1 bis 4, **dadurch gekennzeichnet, dass** die Verbindung der Formel (IV) in einem weiteren Verfahrensschritt in ein 3-(5-Aminotriazolyl)-Sulfid-Derivat der Formel (I) 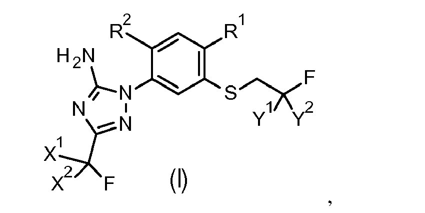 in welcher X¹, X² , Y¹, Y², R¹ und R² die Bedeutungen wie in Anspruch 1 definiert oder in Anspruch 2 definiert oder in Anspruch 3 definiert oder in Anspruch 4 definiert haben, überführt werden.

6. Verfahren nach Anspruch 1 bis 5, **dadurch gekennzeichnet, dass** die Zugabe des Kupferkatalysators bei Raumtemperatur erfolgt.

7. Verfahren nach Anspruch 1 bis 6, **dadurch gekennzeichnet, dass** es sich bei dem Kupferkatalysator um Kupferpulver handelt.

8. Verfahren nach Anspruch 1 bis 6, **dadurch gekennzeichnet, dass** es sich bei dem Kupferkatalysator um ein Kupfersalz in Verbindung mit einem Liganden handelt, wobei das Kupfersalz ausgewählt ist aus der Gruppe bestehend aus CuI, CuCl, Cu₂O, CuOAc, Cu(OAc)₂ und CuCl₂.

9. Verfahren nach Anspruch 8, **dadurch gekennzeichnet, dass** es sich bei dem Ligand um eine Verbindung der Formel (V) handelt 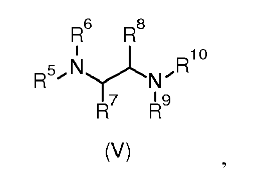 in welcher
R⁵, R⁶, R⁹ und R¹⁰ unabhängig voneinander für Wasserstoff, (C₁-C₆)Alkyl, (C₃-C₆)Cycloalkyl (C₁-C₆)Alkylphenyl oder Phenyl stehen,
R⁷ und R⁸ unabhängig voneinander für Wasserstoff, (C₁-C₆)Alkyl, (C₃-C₆)Cycloalkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
oder gemeinsam für -CH₂-CHR¹¹-CHR¹²-CH₂- stehen, wobei R¹¹ und R¹² unabhängig voneinander für Wasserstoff, (C₁-C₆)Alkyl, (C₃-C₆)Cycloalkyl, (C₁-C₆)Alkylphenyl oder Phenyl stehen.

10. Verfahren nach Anspruch 8 oder 9, **dadurch gekennzeichnet, dass** das molare Verhältnis von Kupfer zu Ligand zwischen 4:1 und 1:50 beträgt.

11. Verfahren nach Anspruch 8 bis 10, **dadurch gekennzeichnet, dass** der Kupfer-Ligand-Komplex in einer Menge von 0,001 bis 20 mol%, bezogen auf den Halogenaromaten der Formel (II), eingesetzt wird.

12. Verfahren nach Anspruch 1 bis 11, **dadurch gekennzeichnet, dass** es sich bei der Lewis-Säure um Fe(acac)₃, FeCl₃, FeCl₂, MgCl₂, NiCl₂, ZnCl₂, Zn(OAc)₂, CoCl₂, MnCl₂, VCl₂, AlCl₃, TiCl₄ oder eine Kombination aus einer oder mehreren dieser Säuren handelt.

13. Verfahren nach Anspruch 1 bis 12, **dadurch gekennzeichnet, dass** die Lewis-Säure oder die Kombination von Lewis-Säuren in einer Gesamtmenge von 0,01 bis 1,0 Äquivalenten, bezogen auf den Halogenaromaten der Formel (II), eingesetzt wird.

14. Verfahren nach Anspruch 1 bis 13, **dadurch gekennzeichnet, dass** es sich bei der Base um NaHCO₃, KHCO₃, Na₂CO₃, K₂CO₃, Cs₂CO₃, K₃PO₄, NaOAc, KOAc, NaOH, KOH, 1,8-Diazabicyclo[5.4.0]undec-7-en (DBU) oder eine Kombination aus einer oder mehreren dieser Basen handelt.

15. Verfahren nach Anspruch 1 bis 14, **dadurch gekennzeichnet, dass** die Base oder die Kombination von Basen in einer Gesamtmenge von 0,1 bis 10,0 Äquivalenten, bezogen auf den Halogenaromaten der Formel (II), eingesetzt wird.

16. Verfahren nach Anspruch 1 bis 15, **dadurch gekennzeichnet, dass** es sich bei dem Lösungsmittel um THF, 1,4-Dioxan, Diethylether, Diglyme, Methyltertbutylether (MTBE), tert-Amyl-methylether (TAME), 2-Methyl-THF, Acetonitril, Butyronitril, Toluol, Xylol, Mesitylen, Ethylacetat, Isopropylacetat, Butylacetat, Pentylacetat, Methylisobutylketon, Alkohole wie Ethylenglycol, Ethylencarbonat, Propylencarbonat, N,N-Dimethylacetamid, N,N-Dimethylformamid, N-Methylpyrrolidon, Halogenkohlenwasserstoffe, aromatische Kohlenwasserstoffe, Chlorkohlenwasserstoffe, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol, 4-Methoxybenzol, fluorierte Aliphate oder Aromaten, Trichlortrifluorethan, Benzotrifluorid, 4-Chlorbenzotrifluorid, Wasser oder deren Gemische handelt.

17. Verbindung der Formel (IIIa) 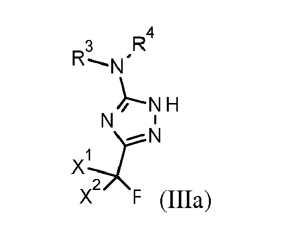 in welcher
X¹ und X² unabhängig voneinander für Fluor, Chlor oder Wasserstoff stehen, und
R³ und R⁴ unabhängig voneinander für Wasserstoff, Trialkylsilyl, Benzyl, *para*-Methoxybenzyl oder gemeinsam für einen Rest #=CHR⁵ stehen, wobei R⁵ für ein ggf. substituiertes Phenyl steht, wobei R³ und R⁴ nicht gleichzeitig Wasserstoff sind.
